Europäisches Patentamt

European Patent Office

Office européen des brevets

(11) Veröffentlichungsnummer: **0 323 535**
**A1**

## (12) EUROPÄISCHE PATENTANMELDUNG

(21) Anmeldenummer: 88100072.3

(22) Anmeldetag: 05.01.88

(51) Int. Cl.⁴ **A61B 5/03**

(43) Veröffentlichungstag der Anmeldung:
12.07.89 Patentblatt 89/28

(84) Benannte Vertragsstaaten:
AT BE CH DE FR GB LI NL

(71) Anmelder: **Hellige GmbH**
**Postfach 728 Heinrich-von-Stephan-Strasse**
**4**
**D-7800 Freiburg im Breisgau(DE)**

(72) Erfinder: **Kronberg, Harald, Dr.**
**Mülhauser Strasse 6**
**D-7813 Staufen(DE)**

(74) Vertreter: **Patentanwälte TER MEER - MÜLLER**
**- STEINMEISTER**
**Mauerkircherstrasse 45**
**D-8000 München 80(DE)**

(54) **Applikationsvorrichtung zur auswechselbaren Implantation von Biosensoren in die Schädelkalotte.**

(57) Eine Applikationsvorrichtung zur auswechselbaren Implantation von Biosensoren, insbesondere von Drucksensoren, für Messungen im Schädelinnenraum bei Kleinkindern ist mit einer Abstützhülse (7) versehen, welche eine axialen Längenausgleich unter Berücksichtigung der geringeren Schädelknochendicke und gleichzeitig eine Führung der Applikationsvorrichtung (5) bzw. des Biosensors (4) gewährleistet.

EP 0 323 535 A1

# Applikationsvorrichtung zur auswechselbaren Implantation von Biosensoren in die Schädelkalotte

Die Erfindung betrifft eine Applikationsvorrichtung zur auswechselbaren Implantation von Biosensoren in die Schädelkalotte gemäß dem Oberbegriff des Patentanspruchs 1.

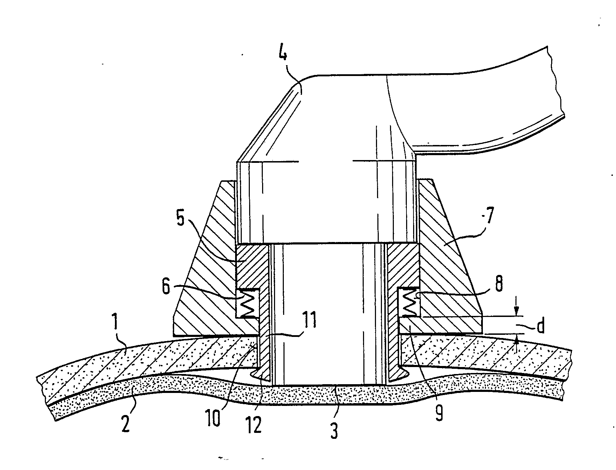

In der EP-A1-0 248 103 ist eine Applikationsvorrichtung der eingangs genannten Art beschrieben, mit der ein Biosensor, insbesondere ein Drucksensor, verkantfrei, mit genau definiertem Tiefenabstand und mit exakt coplanarer Ausrichtung seiner Meßmembran zur Dura mater (harten Hirnhaut, im folgenden kurz "Dura") in eine in die Schädelkalotte eingebrachte Bohrung implantieren läßt. Wie die Fig. 1 bis 4 und die zugehörige Beschreibung der genannten Druckschrift EP-A1-0 248 103 erkennen lassen, wird insbesondere für Schädelinnendruckmessungen vom Chirurgen mittels eines Stufentrepans in den Schädelknochen zunächst ein abgestuftes Loch mit einem näher an der Dura liegenden inneren engeren Durchmesserbereich und einem erweiterten äußeren Durchmesserbereich eingebracht. In diese abgestufte Bohrung wird sodann eine Federbeinhülse eingesetzt, deren untere, zur Schädelinnenseite weisende Federbeine beim Einschieben des Biosensors in die Federbeinhülse oder in vorteilhafter Lösung gemäß der EP-A1-0 248 103 mittels einer zuvor eingesetzten Spreizhülse nach außen gedrückt werden, wobei an den inneren Enden vorgesehene Rastnocken die untere Innenkante am engeren Durchmesserbereich des abgestuften Lochs in der Schädelkalotte untergreifen, um so einen sicheren Bezug für die axial und radial richtige Positionierung des Biosensors zu gewährleisten.

Die Applikationsvorrichtungen für derartige Biosensoren zur Messung des Schädelinnendrucks sowie transdurale oder auch subdurale $pO_2$- oder $pCO_2$-Sensoren oder auch für Stoffwechselsensoren zur Messung des Stoffwechsels im oberen Liquorraum sind in aller Regel auf die durchschnittlichen Schädelknochendicken von Erwachsenen angepaßt, d. h. die axialen Längen von Applikationsvorrichtung einerseits und Meßelementhalterung des Biosensors andererseits sind auf eine Dicke der Schädelknochen von beispielsweise 3 bis 11 mm angepaßt. Es besteht jedoch zunehmend auch ein Bedarf, Messungen mit solchen Biosensoren, insbesondere mit Drucksensoren, bei Kleinkindern vorzunehmen. Die Schädelknochendicke ist bei Kleinkindern noch wesentlich dünner und der Knochen weicher als bei Erwachsenen. Die üblichen Applikationsvorrichtungen und Biosensoren lassen sich also bei Messungen an Kleinkindern nicht oder nur unter Beachtung ganz besonderer Vorsichtsmaßnahmen verwenden.

Der Erfindung liegt die Aufgabe zugrunde, die Applikation von Biosensoren nach dem in der EP-A1-0 248 103 beschriebenen Verfahren auch bei Kleinkindern mit Schädelknochendicken unter 3 mm problemlos zu ermöglichen.

Die Erfindung ist bei einer Applikationsvorrichtung zur auswechselbaren Implantation von Biosensoren in die Schädelkalotte mit einer in ein Loch im Schädelknochen einzusetzenden rohrförmigen Federbeinhülse, deren dem Schädelinneren zugewandte Federbeinenden mit außenseitigen Rastnocken versehen sind, welche zur Fixierung der Federbeinhülse entlang der inneren Umlaufkante des Lochs dienen und deren oberes Ende nach außen abgestuft auf einen größeren Durchmesser verbreitert ist, gekennzeichnet durch eine Abstützhülse mit einer auf den größeren Außendurchmesser der Federbeinhülse bzw. des Biosensors angepaßten Bohrung und einem nach innen vorspringenden, flanschartig umlaufenden Stützrand am dem Schädelknochen zugewandten stirnseitigen Ende der Abstützhülse, mit einer axialen Dicke des Stützrands, welche bei dünneren Schädelknochen einen axialen Längenausgleich zwischen dem größeren Durchmesserbereich und den Federbeinenden der Federbeinhülse gewährleistet.

Die Erfindung und vorteilhafte Einzelheiten werden nachfolgend unter Bezug auf die Zeichnung näher erläutert, deren **einzige Figur** ein Ausführungsbeispiel für eine erfindungsgemäße Abstützhülse und deren vorteilhafte Anwendung für Applikationsvorrichtungen zur auswechselbaren Implantation von Biosensoren in die Schädelkalotte bei Kleinkindern zeigt.

Bei Kleinkindern ist der Schädelknochen 1 noch wesentlich dünner und weicher als bei Erwachsenen. Trotzdem müssen Messungen von Schädelinnendrücken, aber auch andere, bei bestimmten Krankheitsbildern interessante Messungen auf der oder durch die darunterliegende Dura 2 ausgeführt werden können. Ein vom Chirurgen in den Schädelknochen eingebrachtes Loch ist mit Bezugshinweis 10 angegeben. Um insbesondere bei Schädelinnendruckmessungen eine zur Dura 2 coplanare Ausrichtung einer Meßmembran 3 des Drucksensors 4 und ein verkantfreies Einsetzen des Drucksensors 4 an der Meßstelle gewährleisten zu können, wird eine an sich bei spielsweise aus der genannten Druckschrift EP-A1-0 248 103 bekannte Applikationsvorrichtung verwendet, die im wesentlichen aus einer Federbeinhülse 5 besteht, deren Federbeine 11 durch das Loch 10 im Schädelknochen 1 hindurchgesteckt und dann entweder durch den Drucksensor 4 oder in vorteilhafter Weise durch eine (nicht dargestellte) Spreizhülse gemäß der in EP-A1-0 248 103 beschriebenen Art nach außen gedrückt werden, derart, daß endseitige Rastnocken 12 an den Federbeinen 11 der Federbeinhülse 5 die untere umlaufende Innenkante am Loch 10 untergreifen. Die Federbeinhülse 5 stellt so eine sichere Führung für den Drucksensor 4 dar und gewährleistet ein genaue axiale und radiale Führung des Drucksensors 4 bei zur Dura 2 genau coplanarer Ausrichtung der Meßmembran 3. Ein Ringfederelement 6, das beispielsweise aus Siliconkautschuk gefertigt sein kann, bewirkt einen gewissen Axialausgleich zwischen dem schulterartig nach außen springenden größeren Durchmesserbereich der Federbeinhülse 5 und dem umlaufenden Rand an der Bohrung 10 des Knochens 1.

Nun ist jedoch bei Kleinkindern die Dicke des Schädelknochens 1 noch so gering, daß bei Verwendung üblicher Applikationsvorrichtungen und Biosensoren die Federbeinhülse 5 und damit der Biosensor nicht mehr lagestabil in das Bohrloch 10 eingesetzt werden können und möglicherweise zu weit in den Schädelinnenraum eindringen würden. Gemäß der Erfindung wird, um den geschilderten Problemen abzuhelfen, eine Abstützhülse 7 verwendet, deren Axialbohrung 8 auf den größeren oberen Durchmesserbereich der Federbeinhülse 5 bzw. gegebenenfalls auf den oberen Durchmesserbereich des Drucksensors 4 angepaßt ist. Am unteren, dem Schädelknochen 1 zugewandten Ende ist die Abstützhülse 7 mit einem nach innen vorspringenden flanschartigen, umlaufenden Stützrand 9 versehen, dessen Bohrungsdurchmesser auf den normalerweise gewünschten Bohrungsdurchmesser im Schädelknochen in Anpassung auf die zur Verfügung stehenden Applikationsvor richtungen bzw. Biosensoren abgestimmt ist. Die axiale Dikke des umlaufenden Stützrands 9 ist so bemessen, daß ein axialer Längenausgleich zwischen dem oberen größeren Durchmesserbereich der Federbeinhülse 5 unter Berücksichtigung einer durchschnittlich gewünschten Fixierkraft durch das Federelement 6 gewährleistet wird, derart, daß beim Einsetzen der Federbeinhülse 5 durch die Abstützhülse 7 hindurch eine ausreichende Verankerung der Rastnokken 12 an der umlaufenden Innenkante des Lochs 10 erreicht wird.

Wie dargestellt, besteht die Abstützhülse 7 aus einem konisch oder kegelstumpfförmig geformten Körper, beispielsweise aus Titan (wiederverwendbar) oder aus Kunststoff (Wegwerfteil). Die Grundfläche der kegelstumpfförmigen Abstützhülse 7 ruht auf dem Schädelknochen 1, so daß eine bessere Kräfteverteilung beim Einsetzen des Biosensors bzw. der Applikationsvorrichtung gegeben ist.

Mit der erfindungsgemäßen Abstützhülse ist es möglich, übliche Biosensoren und deren Applikationsvorrichtung für Messungen im Schädelinneren auch bei Kleinkindern problemlos zu verwenden.

## Ansprüche

1. Applikationsvorrichtung zur auswechselbaren Implantation von Biosensoren in die Schädelkalotte mit einer in ein Loch (10) im Schädelknochen (1) einzusetzenden rohrförmigen Federbeinhülse (5), deren dem Schädelinneren zugewandte. Federbeinenden mit außenseitigen Rastnocken (12) versehen sind, welche zur Fixierung der Federbeinhülse (5) entlang der inneren Umlaufkante des Lochs (10) dienen und deren oberes Ende nach außen abgestuft auf einen größeren Durchmesser verbreitert ist, **gekennzeichnet durch** eine Abstützhülse (7) mit einer auf den größeren Außendurchmesser der Federbeinhülse (5) bzw. des Biosensors (4) angepaßten Bohrung (8) und einem nach innen vorspringenden flanschartig umlaufenden Stützrand (9) am dem Schädelknochen (1) zugewandten stirnseitigen Ende der Abstützhülse (7), mit einer axialen Dicke (d) des Stützrands (9), welche bei dünneren Schädelknochen einen axialen Längenausgleich zwischen dem größeren Durchmesserbereich und den Federbeinenden der Federbeinhülse (5) gewährleistet.

2. Applikationsvorrichtung nach Anspruch 1, **dadurch gekennzeichnet**, daß die Abstützhülse als konischer (kegelstumpfförmiger) Körper ausgebildet ist, dessen größere Grundfläche im Bereich des Stützrands (9) liegt.

3. Applikationsvorrichtung nach Anspruch 1 oder 2, **dadurch gekennzeichnet**, daß die Abstützhülse (7) aus nichtrostendem Material, insbesondere Titan, hergestellt ist.

4. Applikationsvorrichtung nach Anspruch 1 oder 2, **dadurch gekennzeichnet**, daß die Abstützhülse (7) aus Kunststoff hergestellt ist.

Europäisches
Patentamt

**EUROPÄISCHER RECHERCHENBERICHT**

Nummer der Anmeldung

EP 88 10 0072

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,D | EP-A-0 248 103  (HELLIGE GmbH)<br>* Zusammenfassung; Spalte 4, Zeile 54 - Spalte 6, Zeile 24; Figuren 1-4 *<br>--- | 1 | A 61 B   5/03 |
| A | FR-A-2 274 261  (L'ELECTRONIQUE APPLIQUEE)<br>* Insgesamt *<br>--- | 1 | |
| A | FR-A- 985 553  (A. VENGHIATTIS)<br>* Insgesamt *<br>--- | 1 | |
| A | WO-A-8 701 041  (C. ARVIDSSON)<br>* Zusammenfassung; Seite 6, Zeile 22 - Seite 7, Zeile 15; Seite 8, Zeile 29 - Seite 9, Zeile 19; Figuren 1,3,4 *<br>----- | 1,3,4 | |

| RECHERCHIERTE SACHGEBIETE (Int. Cl.4) |
|---|
| A 61 B<br>A 61 M<br>A 61 N |

Der vorliegende Recherchenbericht wurde für alle Patentansprüche erstellt

| Recherchenort | Abschlußdatum der Recherche | Prüfer |
|---|---|---|
| DEN HAAG | 06-09-1988 | FERRIGNO, A. |

EPO FORM 1503 03.82 (P0403)

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
................................................................
& : Mitglied der gleichen Patentfamilie, übereinstimmendes
    Dokument